# EUROPÄISCHE PATENTSCHRIFT

(11) **EP 2 912 004 B1**
(45) Veröffentlichungstag und Bekanntmachung des Hinweises auf die Patenterteilung: **03.05.2017**
(21) Anmeldenummer: 13783049.3
(22) Anmeldetag: 25.10.2013
(51) Int. Cl.: C07C 41/26, C08F 4/655

(54) **NIEDRIGVISKOSE, KONZENTRIERTE LÖSUNGEN VON ERDALKALIMETALLALKOXIDEN IN APROTISCHEN LÖSUNGSMITTELN UND VERFAHREN ZU DEREN HERSTELLUNG**
LOW-VISCOSITY CONCENTRATED SOLUTIONS OF ALKALINE EARTH METAL ALKOXIDES IN APROTIC SOLVENTS AND PROCESSES FOR PREPARATION THEREOF
SOLUTIONS CONCENTRÉES FAIBLEMENT VISQUEUSES D'ALCOXYDES DE MÉTAUX ALCALINO-TERREUX DANS DES SOLVANTS APROTIQUES ET PROCÉDÉ POUR LES PRÉPARER

(30) Priorität: 25.10.2012 DE 102012219494
(43) Veröffentlichungstag der Anmeldung: 02.09.2015
(73) Patentinhaber: Rockwood Lithium GmbH, 60487 Frankfurt (DE)
(72) Erfinder: WIETELMANN, Ulrich, 61381 Friedrichsdorf (DE); STOLL, Armin, 69502 Hemsbach (DE); KIEFER, Florian, 38640 Goslar (DE); EMMEL, Ute, 65929 Frankfurt am Main (DE)
(74) Vertreter: Uppena, Franz
(86) Internationale Anmeldenummer: PCT/EP2013/072348
(87) Internationale Veröffentlichungsnummer: WO 2014/064233

(56) Entgegenhaltungen:
- EP-A1- 0 244 917
- WO-A1-2010/146122

## Beschreibung

Die Erfindung betrifft niedrigviskose, konzentrierte Lösungen von Erdalkalimetallalkoxiden in aprotischen Lösungsmitteln sowie ein Verfahren zu deren Herstellung.

Magnesiumalkoxide werden u.a. zur Herstellung geträgerter Olefin-Polymerisationskatalysatoren vom Ziegler-Natta - Typ benötigt. Dazu werden beispielsweise unlösliche Alkoxide wie z.B. Magnesiumethoxid in Form sphärischer Partikel eingesetzt, welche durch Reaktion mit Titanchlorid oder einer anderen Titan-Halogenbindungen aufweisenden Verbindung (z. B. Cp₂TiCl₂) in die aktive Form überführt werden (EP 1031580):

Mg(OEt)₂ + Cp₂TiCl₂ → Mg(OEt)₂₋ₓClₓ + Cp₂TiCl₂₋ₓ(OEt)ₓ (x = 0 bis 2)

Eine weitere Möglichkeit, geträgerte Ziegler-Natta - Katalysatoren herzustellen besteht darin, von löslichen Magnesiumalkoxiden auszugehen. Während die meisten Magnesiumalkoholate wie z.B. die Mg-Salze des Methanols, Ethanols, Propanols, Isopropanols, tert-Butanols etc in aprotischen Lösungsmitteln unlöslich sind, erweisen sich die Mg-Verbindungen primärer Alkohole, die eine Verzweigung in 2-Stellung aufweisen, als löslich in Kohlenwasserstoffen (WO 85/02176). So sollen sich beispielsweise die Magnesiumsalze des 2-Methyl-1-pentanols oder des 2-Ethyl-1-hexanols in Konzentrationen von 1,3 mol/l in Cyclohexan lösen. Auch gemischte Mg-alkoxide, d.h. solche mit zwei verschiedenen Alkoxid-Resten Mg(OR¹)(OR²) können kohlenwasserstoff-löslich sein, wenn der korrespondierende Alkohol R¹OH ein in 2-Stellung verzweigter primärer Alkohol und der korrespondierende Alkohol R2OH ein sekundärer Alkohol ist (WO 85/02176).

Nachteilig an Kohlenwasserstoff-Lösungen, die außer Magnesium kein weiteres gelöstes Metall aufweisen, sind die relativ hohen Viskositäten. Weiterhin ist es nicht möglich, solche Lösungen direkt durch Umsetzung von Magnesiummetall mit dem Alkohol im gewünschten Kohlenwasserstoff herzustellen, ohne störende Hilfsmittel zuzugeben. Um eine direkte Umsetzung überhaupt zu ermöglichen, muß das Magnesiummetall aktiviert werden, was durch Anätzen mit lod erreicht werden kann. Aber auch mit dieser Maßnahme ist die Umsetzungsgeschwindigkeit selbst beim Einsatz von hochreaktivem Mg-Pulver noch sehr gering. So wird in (EP 0156512) die Herstellung einer verdünnten Lösung von Magnesium bis(2-ethylhexoxid) in Dodecan unter Verwendung von lod beschrieben. Bei einer Reaktionstemperatur von 145 °C wird eine zehnstündige Reaktionszeit benötigt und das Produkt in Form einer viskosen Lösung erhalten. Eine andere Möglichkeit der Magnesiumaktivierung besteht darin, das Erdalkalimetall mit Trialkylaluminiumverbindungen zu behandeln (WO 2007/026016). Diese Methode hat den Vorteil, dass das Produkt nicht mit lod verunreinigt wird. Allerdings sind die Reaktionsgeschwindigkeiten nicht zufriedenstellend und es werden viskose Produkte erhalten, welche relativ stark mit protischen Verunreinigungen (vor allem freiem Alkohol) verunreinigt sind.

Um die extrem langen Reaktionszeiten zu umgehen, werden Magnesiumalkoholatlösungen deshalb im allgemeinen ausgehend von kommerziell erhältlichen Dialkylmagnesiumverbindungen (R₂Mg) hergestellt. Diese Syntheseroute hat jedoch den Nachteil, dass eine relativ teure Magnesiumquelle (nämlich die R₂Mg-Verbindungen, für deren Herstellung Halogenalkane benötigt werden) verwendet wird. Weiterhin impliziert sie eine Festlegung auf bestimmte Lösungsmittel, nämlich gesättigte Kohlenwasserstoffe: Dialkylmagnesiumverbindungen, beispielsweise Dibutylmagnesium, Butylethylmagnesium und Butyloctylmagnesium sind nämlich nur in gesättigten Kohlenwasserstoffen wie Hexan oder Heptan kommerziell erhältlich. Außerdem entstehen bei der Alkoholyse gemäß

R³R⁴Mg + 2 ROH → Mg(OR)₂ + R³H + R⁴H

unvermeidbar gesättigte Kohlenwasserstoffe (R³H und R⁴H, beispielsweise Butan oder Octan). Eine direkte Herstellung von Magnesiumalkoholaten in reinaromatischen Lösungsmitteln wie Toluol oder Ethylbenzol ist deshalb ausgehend von kommerziell erhältlichen Dialkylmagnesiumverbindungen nicht möglich.

Eine weitere Synthesevariante zur Herstellung löslicher Erdalkalialkoholate besteht in der Umalkoholisierung unlöslicher Erdalkali-Alkoholate, hergestellt aus leicht flüchtigen Alkoholen (beispielsweise Ethanol) mit einem höher siedenden Alkohol, z.B.:

Mg(OR⁵)₂ + 2 ROH → Mg(OR)₂ + 2 R⁵OH

Nachteilig ist der relativ hohe, kostenintensive Aufwand dieser Methode: das Alkoholat Mg(OR⁵)₂ muss zunächst aus dem flüchtigem Alkohol R⁵OH und Magnesiummetall hergestellt und isoliert werden, dann mit einem weniger flüchtigen Alkohol, beispielsweise 2-Ethylhexanol, umgesetzt werden und dann muss der flüchtigere Alkohol R⁵OH z.B. destillativ entfernt werden.

Die relativ hohe Viskosität von Magnesiumalkoxidlösungen wird durch Assoziationsphänomene verursacht. Es ist bekannt, dass die Viskosität durch Zugabe von Alkylaluminiumverbindungen reduziert werden kann. Das bevorzugte Verhältnis zwischen Alkylaluminiumverbindung und Mg-Alkoholat liegt zwischen 0,001:1 und 1:1, mehr bevorzugt 0,01 bis 0,1:1 und ganz besonders bevorzugt 0,03 bis 0,05:1 (US 6,734,134).

Aus der WO2010/146122 schließlich ist bekannt, gemischte Erdalkalialkoxidverbindungen M(OCH₂R⁶)₂₋ₓ(OR⁷)ₓ in Mischung mit einer Aluminiumverbindung Al(OCH₂R⁶)_{3-y}(OR⁷)_{y} in aprotischen Lösungsmitteln ausgehend von einem Erdalkalimetall und zwei verschiedenen Alkoholen herzustellen. Dabei ist M ein Erdalkalimetall ausgewählt aus Mg, Ca, Ba, Sr;
OCH₂R⁶ ein Alkoxidrest bestehend aus mindestens 3 und höchstens 40 C-Atomen mit einer Verzweigung in 2-Position bezogen auf die O-Funktion, also R⁶ = -CHR⁸R⁹ mit R⁸, R⁹ = unabhängig voneinander Alkylreste C₁ - C₁₈;
R⁷ ein Alkylrest mit 2-15 C-Atomen, der entweder linear ist oder eine Verzweigung ≥ der 3-Position (bezogen auf die O-Funktion) aufweist
und die Summe von x und y eine Zahl zwischen 0,01 und 0,8, bevorzugt 0,02 und 0,3 und besonders bevorzugt 0,03 und 0,2.

Die mit Hilfe dieses Verfahrens hergestellten Produktlösungen weisen zwar relativ hohe Konzentrationen an Erdalkalialkoxidverbindungen auf (d.h. cMg > 0,5 mol/kg), jedoch sind die Viskositäten mit typisch ≥ 1.000 cP noch unbefriedigend hoch (siehe auch Vergleichsbeispiel in vorliegender Schrift).

Es ist ein einfaches Verfahren gesucht, welches ausgehend von einer billigen Magnesiumquelle und mit hoher Raum/Zeit-Ausbeute wenig viskose, konzentrierte, d.h. ≥ 0,5, bevorzugt ≥ 1,0 mol/kg Lösungen eines Magnesiumalkoholates in aprotischen Lösungsmitteln, bevorzugt aliphatischen oder aromatischen Kohlenwasserstoffen, liefert. Weiterhin sollen die gewünschten Produkte möglichst geringe Gehalte an störenden Verunreinigungen wie z.B. lod und protischen Stoffen wie Alkoholen und Wasser aufweisen, so dass sie für die Herstellung von Ziegler-Natta-Katalysatoren geeignet sind.

Die Aufgabe wird dadurch gelöst, dass gemischte Erdalkalialkoxidverbindungen M(OCH₂R⁶)_{2-a-b}(OR⁷)ₐ[O(CHR⁸)ₙOR⁹]_{b} in Mischung mit einer Aluminiumverbindung Al(OCH₂R⁶)_{3-c-d}(OR⁷)_{c}[O(CHR⁸)ₙOR⁹]_{d} in aprotischen Lösungsmitteln bereitgestellt werden. Dabei ist
M ein Erdalkalimetall ausgewählt aus Mg, Ca, Ba, Sr;
OCH₂R⁶ ein Alkoxidrest bestehend aus mindestens 3 und höchstens 40 C-Atomen mit einer Verzweigung in 2-Position bezogen auf die O-Funktion, also R⁶ = - CHR¹⁰R¹¹ mit R¹⁰, R¹¹ = unabhängig voneinander Alkylreste C₁ - C₁₈;
R⁷ ein Alkylrest mit 2-15 C-Atomen, der entweder linear ist oder eine Verzweigung ≥ der 3-Position (bezogen auf die O-Funktion) aufweist;
R⁸ ein Alkylrest mit 1-6 C-Atomen, der entweder linear ist oder eine Verzweigung ≥ der 3-Position (bezogen auf die O-Funktion) aufweist;
R⁹ ein Alkylrest mit 2-15 C-Atomen, der entweder linear ist oder eine Verzweigung aufweist;
n = eine ganze Zahl zwischen 1 und 4 und
a + b ≤ 2 sowie c + d ≤ 3 und a sowie c können beliebige Werte von 0,01-0,8 und b und d können beliebige Werte von 0,1-1,99 einnehmen, wobei der Gehalt an gelöstem Aluminium bezogen auf gelöstes Erdalkalimetall im Bereich zwischen 0,2 und etwa 20 mol % liegt. Das aprotische Lösungsmittel ist oder enthält entweder einen oder mehrere aliphatische Verbindungen mit 5 bis 20 C-Atomen, wobei sowohl zyklische als auch offenkettige Verbindungen möglich sind. Bevorzugt sind: Cyclohexan, Methylcyclohexan, Hexan, Heptan, Octan, Nonan, Dekan, Dodecan, Dekalin sowie handelsübliche Siedeschnitte (Benzinfraktionen).

Das aprotische Lösungsmittel kann weiterhin Aromaten enthalten oder daraus bestehen. Bevorzugt sind: Benzol, Toluol, Ethylbenzol, Xylole sowie Cumol.

In einer weiteren Ausführungsform der Erfindung kann die erfindungsgemäße Erdalkalialkoxidlösung noch polare, aprotische Lösungsmittel wie z.B. Ether oder tertiäre Amine enthalten.

Der in 2-Position verzweigte Alkohol (HOCH₂R⁶) ist besonders bevorzugt ausgewählt aus der Gruppe bestehend aus: Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder einer beliebigen Mischung aus mindestens zwei der aufgeführten Alkohole. Der primäre Alkohol (HOR⁷) ist bevorzugt ausgewählt aus der Gruppe bestehend aus: Ethanol, Propanol, Butanol, Pentanol, Hexanol, Octanol, Decanol, Dodekanol, 3-Methylbutan-1-ol oder einer beliebigen Mischung aus mindestens zwei der aufgeführten Alkohole. Der eine Alkoxyfunktion enthaltende Alkohol HO(CHR⁸)ₙOR⁹ ist bevorzugt ein C₂-C₄ - Glykolmonoether, beispielsweise 2-Ethoxyethanol, 3-Ethoxy-1-propanol, 3-Ethoxy-1-butanol, 2-(2-Ethylhexoxy)ethanol, 2-Butoxyethanol, 2-Hexyloxyethanol sowie 1,3-Propylenglycolmonobutylether oder eine beliebige Mischung aus mindestens zwei der aufgeführten Alkohole.

Die erfindungsgemäßen Produkte werden im allgemeinen wie folgt hergestellt: handelsübliches Erdalkalimetall, bevorzugt Magnesiummetall, dieses bevorzugt in Pulver-, Granulat- oder Spanform, wird in einem wasserfreien aprotischen Lösungsmittel, bevorzugt aromatischen oder aliphatischen Kohlenwasserstoffen in einem inertisierten, d.h. getrockneten und mit Schutzgas (Stickstoff oder Argon) versehenem Rührwerksbehälter vorgelegt. Dann wird eine Alkylaluminiumverbindung, z.B. Trialkylaluminium wie Triethylaluminium, Tributylaluminium, ein Alkylaluminiumhydrid wie Dibutylaluminiumhydrid, ein Alkylaluminiumhalogenid wie Dibutylaluminiumchlorid oder eine Alkoxyaluminiumverbindung wie beispielsweise Diethylaluminiumethoxid zugegeben und ca. 5 Minuten bis etwa zwei Stunden bei ca. 20 bis 180 °C, bevorzugt 40 bis 120 °C gerührt. Die optimale Menge an Alkylaluminiumverbindung richtet sich nach der Erdalkalimetallqualität, insbesondere der Magnesiumqualität, sowie der im nächsten Schritt zugegebenen Menge an Alkoholen. Im Allgemeinen liegt das Molverhältnis Alkylaluminiumverbindung zu den Alkoholen zwischen 0,0001 und 0,1 zu 1, bevorzugt zwischen 0,005 und 0,04 zu 1.

Dann werden ein eine Alkoxyfunktion enthaltender Alkohol HO(CHR⁸)ₙOR⁹, ein verzweigter Alkohol HOCH₂R⁶ sowie ein unverzweigter oder eine Verzweigung ≥ der 3-Position aufweisender primärer Alkohol mit 2-15 C-Atomen (HOR⁷) zugegeben. Die Zugabe kann entweder nacheinander in beliebiger Reihenfolge oder in Mischung erfolgen. Bevorzugt wird zunächst der primäre Alkohol R⁷OH zugegeben, dann erst die beiden anderen Alkohole. Die Zugabe kann bei Temperaturen zwischen ca. 0 und 180 °C erfolgen, bevorzugt zwischen etwa 40 und 140 °C. Ganz besonders bevorzugt erfolgt sie am Siedepunkt des verwendeten Lösungsmittels, also z.B. im Falle von Toluol bei etwa 110 °C. Die Reaktionszeit richtet sich nach der Reaktivität des verwendeten Erdalkalimetalls, insbesondere des Magnesiums, sowie der Acidität des verwendeten Alkohols, dem stöchiometrischen Verhältnis zwischen Erdalkalimetall, insbesondere Magnesium, und den Alkoholen und der Reaktionstemperatur sowie nach den Anforderungen an das Endprodukt, insbesondere dem zulässigen oder gewünschten Restgehalt an freiem Alkohol. Wird das Erdalkalimetall, insbesondere das Magnesium, im Überschuss eingesetzt (bevorzugt 1 bis 300 %, besonders bevorzugt 10 bis 100 %), reichen bei der Rückflussfahrweise ca. 1 bis 6 Stunden Reaktionszeit aus. Produkte mit einem gewünschten, höheren Restalkoholgehalt werden demgegenüber mit überschüssigem Alkohol, beispielsweise 5 - 20 mol%, hergestellt.

Nach Beendigung der Reaktion, erkennbar am Versiegen des Wasserstoffstromes, wird gegebenenfalls überschüssiges Erdalkalimetall, insbesondere Magnesiummetall, von der gewünschten Produktlösung abgetrennt. Dies kann dekantativ, per Filtration oder Zentrifugation erfolgen.

Die nach erfindungsgemäßem Verfahren hergestellten Produkte sind überraschenderweise trotz hoher Erdalkalimetall-Konzentration von ≥ 0,5 mol/kg, bevorzugt ≥ 1,0 mol/kg sehr wenig viskos und sie haben einen geringen Gehalt an protischen Verunreinigungen. Die Erdalkalimetall - Konzentrationen liegen bevorzugt im Bereich von ca. 0,4 bis 1,6 mmol/g, besonders bevorzugt zwischen 0,7 und 1,4 mmol/g. Die bei Raumtemperatur gemessenen Viskositäten liegen im allgemeinen unter 300 cP bevorzugt unter 200 cP, besonders bevorzugt unter 100 cP. Die Gehalte an protischen Verunreinigungen liegen, bezogen auf gelöstes Erdalkalielement, im allgemeinen zwischen 0,1 und 40 mol%, bevorzugt 1 und 30 mol%.

Der Gehalt an gelöstem Aluminium liegt bezogen auf gelöstes Erdalkalimetall im Bereich zwischen ca. 0,2 und ca. 20 mol %, bevorzugt zwischen 1 und 8 mol%. Der Anteil des unverzweigten oder eine Verzweigung ≥ der 3-Position aufweisenden primären Alkohols mit 2-15 C-Atomen HOR⁷ an der gesamten Alkoholmenge liegt zwischen 0,5 und 40 mol%, bevorzugt zwischen 1 und 20 mol%, besonders bevorzugt 1,5 und 10 mol%. Der Anteil des eine Alkoxyfunktion enthaltenden Alkohols HO(CHR⁸)ₙOR⁹ an der Gesamtalkoholmenge beträgt zwischen 5 und 99,5mol%, bevorzugt 10 - 99 mol%. Bevorzugt enthält die erfindungsgemäße Produktlösung 0,1 bis 80 mol%, besonders bevorzugt 1 bis 40 mol% freien Alkohol, bezogen auf in Lösung befindliches Erdalkalimetall.

Die erfindungsgemäßen Produkte werden für die Herstellung von Polymerisationskatalysatoren, insbesondere heterogenisierte Polyolefinkatalysatoren vom Ziegler-Natta-Typ verwendet. Ferner können Sie in der organischen Synthese, beispielsweise als Basen eingesetzt werden.

### Beispiele

Alle Umsetzungen wurden in trockenen, mit Argon inertisierten Glasapparaturen vorgenommen. Es wurden handelsübliche Magnesiumspäne eingesetzt. Die Konzentrationen an Mg und Al wurden mittels ICP (induktiv gekoppeltes Plasma) gemessen. Der Gehalt an protischen Verunreinigungen wurde gasvolumetrisch durch Umsetzung mit ca. 1 %iger LiAlH₄-Lösung in THF unter Eiskühlung bestimmt.

Die bei der Synthese gemessenen Gasmengen übersteigen in der Regel den Erwartungswert, da der Reaktionswasserstoff lösemitteldampfgesättigt ist und aus der eingesetzten Trialkylaluminiumverbindung gasförmige Kohlenwasserstoffe (z.B. Ethan aus Triethylaluminium) freigesetzt werden.

### Beispiel 1: Herstellung von Magnesium bis(2-butoxyethanolat) in Toluol/Heptanmischung mit Zusatz von 3,5mol% Ethanol (bezogen auf Gesamtalkoholmenge)

In einem 0,5 L-Doppelmantelglasreaktor mit Rückflusskühler und Tropftrichter wurden 13,5 g Magnesiumspäne 216 g Toluol und 90 g Heptan vorgelegt. Dann wurden 7,6 g einer 25%igen Lösung von Triethylaluminium in Toluol zugespritzt und zum Siedepunkt aufgeheizt. Es wurden 2,3 g Ethanol und 163 g 2-Butoxyethanol innerhalb von 120 Minuten zugetropft. Dabei entwickelten sich 15,1 L Gas (107% d.Th.). Nach Dosierende wurde der Reaktorinhalt noch weitere 90 Minuten refluxiert, wobei sich weitere 0,3 L Gas entwickelten.

Nach Abkühlung auf ca. 80 °C wurde die hellgraue Suspension abgehebert und filtriert. Es wurden 425 g einer nichtviskosen Flüssigkeit mit einem Magnesiumgehalt von 1,26 mmol/g (entsprechend einem Umsatz von 102 % d. Th.) erhalten. Die Produktlösung enthielt weiterhin 0,035 mmol/g Aluminium und sie wies einen Gehalt an protischen Verunreinigungen von 0,23 mmol/g auf.
Ausbeute: 96 % d. Th.
Viskosität (Brookfield): 10 cP

### Vergleichsbeispiel 1: Herstellung von Magnesium bis(2-butoxyethanolat) in Toluol/Heptanmischung ohne Zusatz eines primären unverzweigten oder eine Verzweigung ≥ der 3-Position aufweisenden primären Alkohols mit 2-15 C-Atomen HOR⁷

In einem 0,5 L-Doppelmantelglasreaktor mit Rückflusskühler und Tropftrichter wurden 14,7 g Magnesiumspäne, 215 g Toluol und 90 g Heptan vorgelegt. Dann wurden 7,6 g einer 25%igen Lösung von Triethylaluminium in Toluol zugespritzt und zum Siedepunkt aufgeheizt: Bei Innentemperaturen um 104°C wurden 163,5 g 2-Butoxyethanol (Ethylenglykolmonobutylether) innerhalb von 90 Minuten zugetropft.

Es entwickelten sich 3,4 L Gas (24% d.Th.) und die Lösung wurde zunehmend viskoser und dunkel (fast schwarz). Nach Dosierende wurde der Reaktorinhalt noch weitere 4 h refluxiert, wobei sich weitere 0,6 L Gas entwickelten.

Nach Abkühlung auf ca. 80 °C wurde die dunkelgraue Suspension abgehebert und filtriert. Es wurden 411 g einer viskosen Flüssigkeit mit einem Magnesiumgehalt von 0,27 mmol/g (entsprechend einem Umsatz von 22 % d. Th.) erhalten. Die Produktlösung enthielt weiterhin 0,034 mmol/g Aluminium und sie wies einen Gehalt an protischen Verunreinigungen von 2,30 mmol/g auf.
Ausbeute: 18 % d.Th.

### Beispiel 2: Herstellung von Magnesium bis(2-(2-Ethylhexoxy)ethanolat) in Toluol mit Zusatz von 1,5mol% Ethanol (bezogen auf Gesamtalkoholeinsatz)

In einem 0,5 L-Doppelmantelglasreaktor mit Rückflusskühler und Tropftrichter wurden 14,7 g Magnesiumspäne und 304 g Toluol vorgelegt. Dann wurden 7,6 g einer 25%igen Lösung von Triethylaluminium in Toluol zugespritzt und zum Siedepunkt aufgeheizt. Es wurden 0,97 g Ethanol und 236 g 2-(2-Ethylhexoxy)ethanol innerhalb von 120 min zugetropft. Dabei entwickelten sich 12,5 L Gas (86% d.Th.). Nach Dosierende wurde der Reaktorinhalt noch weitere 4 h refluxiert, wobei sich weitere 2,4 L Gas ohne Aufschäumen entwickelten.

Nach Abkühlung auf etwa 80 °C wurde die leicht graue Suspension filtriert. Es wurden 539 g einer fast klaren Flüssigkeit mit einem Magnesiumgehalt von 1,11 mmol/g (entsprechend einem Umsatz von 103 % d. Th.) erhalten. Die Produktlösung enthielt weiterhin 0,032 mmol/g Aluminium und sie wies einen Gehalt an protischen Verunreinigungen von 0,030 mmol/g auf.
Ausbeute: 99% d. Th.
Viskosität (Brookfield): 10 cP
Vergleichsbeispiel 2: Herstellversuch von Magnesium bis(2-(2-Ethylhexoxy)ethanolat) in Toluol ohne Zusatz eines primären unverzweigten oder eine Verzweigung ≥ der 3-Position aufweisenden primären Alkohols mit 2-15 C-Atomen HOR⁷

In einem 0,5 L-Doppelmantelglasreaktor mit Rückflusskühler und Tropftrichter wurden 14,7 g Magnesiumspäne und 305 g Toluol vorgelegt. Dann wurden 7,6 g einer 25%igen Lösung von Triethylaluminium in Toluol zugespritzt und zum Siedepunkt aufgeheizt. Es wurden 240 g 2-(2-Ethylhexoxy)ethanol innerhalb von 120 min zugetropft. Dabei entwickelten sich 1,2 L Gas (8% d.Th.). Nach Dosierende wurde der Reaktorinhalt noch weitere 4 h refluxiert, wobei sich kein weiteres Gas entwickelte.

Nach Abkühlung auf ca. 80 °C wurde die leicht graue Suspension filtriert. Es wurden 545 g einer fast klaren Flüssigkeit mit einem Magnesiumgehalt von < 0,01 mmol/g (entsprechend einem Umsatz von 0 % d. Th.) erhalten. Die Produktlösung enthielt weiterhin 0,032 mmol/g Aluminium und sie wies einen Gehalt an protischen Verunreinigungen von 2,50 mmol/g auf.
Ausabeute: 0% d.Th.

### Vergleichsbeispiel 3: Herstellung von Magnesium-bis(2-ethylhexanolat) in Toluol/Heptan mit Zusatz von 4 mol% Ethanol in Abwesenheit eines eine Alkoxyfunktion enthaltenden Alkohols HO(CHR⁸)ₙOR⁹

In einem 0,5 L-Doppelmantelglasreaktor mit Rückflusskühler und Tropftrichter wurden 18,9 g Magnesiumspäne und 443 g Toluol sowie 40 g Heptan vorgelegt. Dann wurden 9,6 g einer 25%igen Lösung von Triethylaluminium in Toluol zugespritzt und zum Siedepunkt aufgeheizt. Danach wurden 3,11 g Ethanol und 215 g 2-Ethylhexanol innerhalb von 2 Stunden zugetropft. Es entwickelten sich 14,7 L Gas (79% d.Th.). Nach Dosierende wurde der Reaktorinhalt noch weitere 270 Minuten refluxiert, wobei sich weitere 2,9 L Gas ohne Aufschäumen entwickelten (insgesamt 17,6 L, 95% d.Th.).

Nach Abkühlung auf ca. 80 °C wurde die Reaktionsmischung abgehebert und filtriert. Es wurden 615 g einer leicht grauen, klaren Flüssigkeit mit einem Magnesiumgehalt von 1,24 mmol/g (entsprechend einem Umsatz von 103 % d. Th.) erhalten. Die Produktlösung enthielt weiterhin 0,033 mmol/g Aluminium und sie wies einen Gehalt an protischen Verunreinigungen von 0,25 mmol/g auf.
Ausbeute: 98% d.Th.
Viskosität (Brookfield): 3.700 cP.

Beispiel 3: Herstellung einer gemischten Magnesium-bis(2-ethylhexanolat)/ Magnesium bis(2-butoxyethanolat)-Lösung in Toluol/Heptan mit Zusatz von 4 mol% Ethanol (bezogen auf Gesamtalkoholeinsatz)

In einem 0,5 L-Doppelmantelglasreaktor mit Rückflusskühler und Tropftrichter wurden 18,4 g Magnesiumspäne und 443 g Toluol sowie 40 g Heptan vorgelegt. Dann wurden 9,6 g einer 25%igen Lösung von Triethylaluminium in Toluol zugespritzt und zum Siedepunkt aufgeheizt. Danach wurden 3,0 g Ethanol und ein Gemisch aus 108 g 2-Ethylhexanol und 97,5 g 2-Butoxyethanol innerhalb von 2 Stunden zugetropft. Es entwickelten sich 17,2 L Gas (93% d.Th.). Nach Dosierende wurde der Reaktorinhalt noch weitere 120 Minuten refluxiert, wobei sich weitere 1,8 L Gas entwickelten (insgesamt 19,0 L, 103% d.Th.).

Nach Abkühlung auf ca. 80 °C wurde die Reaktionsmischung abgehebert und filtriert. Es wurden 602 g einer leicht grauen, klaren Flüssigkeit mit einem Magnesiumgehalt von 1,26 mmol/g (entsprechend einem Umsatz von 104 % d. Th.) erhalten. Die Produktlösung enthielt weiterhin 0,035 mmol/g Aluminium und sie wies einen Gehalt an protischen Verunreinigungen von 0,23 mmol/g auf.
Ausb.: 98% d. Th.
Viskosität (Brookfield): 80 cP.

Die Vergleichsbeispiele 1 und 2 erfolgten nach der technischen Lehre von WO 2007/026016A1, d.h. das Magnesium wurde mit Trialkylaluminiumlösungen aktiviert, und die Umsetzungen mit dem eine Alkoxyfunktion enthaltenden Alkohol HO(CHR⁸)ₙOR⁹ erfolgten am Siedepunkt.

Beim Einsatz von 2-Butoxyethanol wird in Toluol/Heptan in Abwesenheit eines primären unverzweigten oder eine Verzweigung ≥ der 3-Position aufweisenden primären Alkohols mit 2-15 C-Atomen HOR⁷ nach 6-stündiger Reaktionszeit nur etwa 18 % d.Th. des gewünschten Magnesiumalkoholats erhalten (Vergleichsbeispiel 1). Es wurde eine starke Viskositätszunahme beobachtet. Das Verfahrensprodukt wies noch einen extrem hohen Gehalt an protischen Verunreinigungen auf: 2,30 mmol/g entsprechend 370 mol% bezogen auf gelöstes Magnesium. In Gegenwart von 3,5 mol % Ethanol wurde bei einer verkürzten Reaktionszeit von 3,5 h das Zielprodukt mit 96% Ausbeute erhalten (Beispiel 1). Demzufolge war der Gehalt an protischen Verunreinigungen ganz deutlich auf nur noch 18 mol% zurückgegangen Die Produktviskosität war trotz sehr hoher Produktkonzentration außerordentlich gering (10 cP).

Beispiel 2 und Vergleichsbeispiel zeigen die Ergebnisse beim Einsatz eines längerkettigen alkoxysubstituierten Alkohols, dem 2-(2-Ethylhexoxy)ethanol. In diesem Falle konnte ohne Einsatz eines primären unverzweigten oder eine Verzweigung ≥ der 3-Position aufweisenden primären Alkohols mit 2-15 C-Atomen HOR⁷ überhaupt keine Umsetzung initiiert werden, während bei Verwendung von 1,5 mol% Ethanol eine hochkonzentrierte, nichtviskose Lösung von Magnesium bis(2-(2-Ethylhexoxy)ethanolat) mit 99% Ausbeute erhalten wurde.

In Vergleichsbeispiel 3 wird unter Befolgung der technischen Lehre aus WO2010/146122 ohne Einsatz eines eine Alkoxyfunktion enthaltenden Alkohols HO(CHR⁸)ₙOR⁹ gearbeitet und eine hochkonzentrierte Lösung von Magnesium bis(2-ethylhexanolat) in Toluol/Heptan hergestellt. Die Ausbeute und der Gehalt an protischen Verunreinigungen liegen zwar im gewünschten Bereich, jedoch ist die Viskosität mit 3.700 cP extrem hoch.

Im letzten Beispiel 3 wird ein Gemisch aus drei verschiedenen Alkoholen eingesetzt. In diesem Fall werden gleiche molare Mengen 2-Ethylhexanol und der eine Alkoxyfunktion enthaltende Alkohol 2-Butoxyethanol verwendet. Beim Einsatz von 4 mol % Ethanol wird die gewünschte gemischte Magnesium-bis(2-ethylhexanolat)/ Magnesium bis(2-butoxyethanolat) enthaltende Lösung mit sehr guter Ausbeute erhalten. Die Viskosität ist mit 80 cP vergleichsweise sehr niedrig.

## Patentansprüche

1. Lösungen von gemischten Erdalkalialkoxidverbindungen M(OCH₂R⁶)_{2-a-b}(OR⁷)ₐ[O(CHR⁸)ₙOR⁹]_{b} in Mischung mit einer Aluminiumverbindung Al(OCH₂R⁶)_{3-c-d}(OR⁷)_{c}[O(CHR⁸)ₙOR⁹]_{d} in aprotischen Lösungsmitteln, wobei
• M ein Erdalkalimetall ausgewählt aus Mg, Ca, Ba, Sr;
• OCH₂R⁶ ein Alkoxidrest bestehend aus mindestens 3 und höchstens 40 C-Atomen mit einer Verzweigung in 2-Position bezogen auf die O-Funktion, also R⁶ = -CHR¹⁰R¹¹ mit R¹⁰, R¹¹ = unabhängig voneinander Alkylreste C₁ - C₁₈;
• R⁷ ein Alkylrest mit 2-15 C-Atomen, der entweder linear ist oder eine Verzweigung ≥ der 3-Position (bezogen auf die O-Funktion) aufweist;
• R⁸ ein Alkylrest mit 1-6 C-Atomen, der entweder linear ist oder eine Verzweigung ≥ der 3-Position (bezogen auf die O-Funktion) aufweist;
• R⁹ ein Alkylrest mit 2-15 C-Atomen, der entweder linear ist oder eine Verzweigung aufweist;
• n = eine ganze Zahl zwischen 1 und 4 ist und
• a + b ≤ 2 sowie c + d ≤ 3 und a sowie c beliebige Werte von 0,01-0,8 und b und d beliebige Werte von 0,1-1,99 einnehmen können, wobei der Gehalt an gelöstem Aluminium bezogen auf gelöstes Erdalkalimetall im Bereich zwischen 0,2 und etwa 20 mol % liegt.

2. Lösungen nach Anspruch 1, **dadurch gekennzeichnet, dass** die Erdalkalimetallkonzentration bevorzugt im Bereich von 0,4 bis 1,6, besonders bevorzugt zwischen 0,7 bis 1,4 mmol/g liegt.

3. Lösungen nach Anspruch 1 oder 2, **dadurch gekennzeichnet, dass** die Raumtemperatur-Viskositäten ≤ 300 cP, bevorzugt ≤ 200 cP, besonders bevorzugt ≤ 100 cP liegen.

4. Lösungen nach Anspruch 1 bis 3, **dadurch gekennzeichnet, dass** die Gehalte an protischen Verunreinigungen, bezogen auf gelöstes Erdalkalielement, im allgemeinen zwischen 0,1 und 40 mol%, bevorzugt 1 und 30 mol% liegen.

5. Lösungen nach Anspruch 1 bis 4, **dadurch gekennzeichnet, dass** der Gehalt an gelöstem Aluminium bezogen auf gelöstes Erdalkalimetall im Bereich zwischen 1 und 8 mol% liegt.

6. Lösungen nach Anspruch 1 bis 5, **dadurch gekennzeichnet, dass** als eine Alkoxyfunktion enthaltender Alkohol HO(CHR⁸)ₙOR⁹ bevorzugt ein C₂-C₄ - Glykolmonoether, beispielsweise 2-Ethoxyethanol, 3-Ethoxy-1-propanol, 3-Ethoxy-1-butanol, 2-(2-Ethylhexoxy)ethanol, 2-Butoxyethanol, 2-Hexyloxyethanol sowie 1,3-Propylenglycolmonobutylether oder einer beliebigen Mischung aus mindestens zwei der aufgeführten Alkohole eingesetzt wird.

7. Lösungen nach Anspruch 1 bis 6, **dadurch gekennzeichnet, dass** sie 0,1 bis 80 mol%, besonders bevorzugt 1 bis 40 mol% freien Alkohol, bezogen auf in Lösung befindliches Erdalkalimetall enthalten.

8. Verfahren zur Herstellung von gemischten Erdalkalialkoxidverbindungen M(OCH₂R⁶)₂₋ₓ(OR⁷)ₓ in aprotischen Lösungsmitteln, **dadurch gekennzeichnet, dass** ein mittels Alkylaluminiumverbindungen aktiviertes Erdalkalimetall in einem aprotischen Lösungsmittel mit einem eine Alkoxyfunktion enthaltenden Alkohol HO(CHR⁸)ₙOR⁹ sowie einem unverzweigten oder eine Verzweigung ≥ der 3-Position aufweisenden primären Alkohol mit 2-15 C-Atomen HOR⁷ und gegebenenfalls einem in 2-Stellung verzweigten primären Alkohol umgesetzt wird.

9. Verfahren nach Anspruch 8, **dadurch gekennzeichnet, dass** als Alkylaluminiumverbindung Trialkyl-, Alkyl-Alkoxy- und/oder Alkyl-Halogenid-Verbindungen eingesetzt werden.

10. Verfahren nach Anspruch 8 oder 9, **dadurch gekennzeichnet, dass** als Lösemittel Kohlenwasserstoffe eingesetzt werden, wobei entweder aliphatische Lösemittel ausgewählt aus der Gruppe bestehend aus Cyclohexan, Methylcyclohexan, Hexan, Heptan, Octan, Nonan, Dekan, Dodecan, Dekalin sowie handelsübliche Siedeschnitte (Benzinfraktionen) oder aromatische Lösemittel ausgewählt aus der Gruppe bestehend aus Benzol, Toluol, Ethylbenzol, Xylolen sowie Cumol verwendet werden.

11. Verfahren nach Anspruch 8 bis 10, **dadurch gekennzeichnet, dass** die Umsetzung bei Temperaturen zwischen etwa 0 und 180 °C erfolgt, bevorzugt zwischen etwa 40 und 140 °C, insbesondere bei der Siede-Temperatur des Lösemittels.

12. Verfahren nach Anspruch 8 bis 11, **dadurch gekennzeichnet, dass** als eine Alkoxyfunktion enthaltender Alkohol HO(CHR⁸)ₙOR⁹ bevorzugt ein C₂-C₄ - Glykolmonoether, beispielsweise 2-Ethoxyethanol, 3-Ethoxy-1-propanol, 3-Ethoxy-1-butanol, 2-(2-Ethylhexoxy)ethanol, 2-Butoxyethanol, 2-Hexyloxyethanol sowie 1,3-Propylenglycolmonobutylether oder einer beliebigen Mischung aus mindestens zwei der aufgeführten Alkohole; als in 2-Stellung verzweigter Alkohol (HOCH₂R⁶) Isobutanol, 2-Methyl-1-pentanol, 2-Ethyl-1-butanol, 2-Ethyl-1-pentanol, 2-Ethyl-4-methyl-1-pentanol, 2-Propyl-1-heptanol, 2-Methyl-1-hexanol, 2-Ethylhexanol und 2-Ethyl-5-methyl-1-octanol oder eine beliebige Mischung aus mindestens zwei der aufgeführten Alkohole und als unverzweigter oder eine Verzweigung ≥ der 3-Position aufweisender primärer Alkohol mit 2-15 C-Atomen (HOR⁷) Ethanol, Propanol, Butanol, Pentanol, Hexanol, Octanol, Decanol, Dodekanol, 3-Methylbutan-1-ol oder eine beliebige Mischung aus mindestens zwei der aufgeführten Alkohole eingesetzt werden.

13. Verfahren nach Anspruch 8 bis 12, **dadurch gekennzeichnet, dass** zunächst der Alkohol (HOR⁷), dann erst der eine Alkoxyfunktion enthaltende Alkohol HO(CHR⁸)ₙOR⁹ und der in 2-Stellung verzweigte Alkohol (HOCH₂R⁶) zugegeben wird.

## Claims

1. Solutions of mixed alkaline earth alkoxide compounds M(OCH₂R⁶)_{2-a-b}(OR⁷)ₐ[O(CHR⁸)ₙOR⁹]_{b} in a mixture with an aluminium compound Al (OCH₂R⁶)_{3-c-d} (OR⁷)_{c}[O(CHR⁸)ₙOR⁹]_{d} in aprotic solvents, wherein
• M is an alkaline earth metal selected from Mg, Ca, Ba, Sr;
• OCH₂R⁶ is an alkoxide radical consisting of at least 3 and at most 40 C-atoms having a branch in the 2-position relative to the O-function, that is, R⁶ = -CHR¹⁰R¹¹ with R¹⁰, R¹¹ = independently of each other alkyl radicals C₁-C₁₈;
• R⁷ is an alkyl radical with 2-15 C-atoms which is either linear or has a branch ≥ the 3-position (relative to the O-function);
• R⁸ is an alkyl radical with 1-16 C-atoms which is either linear or has a branch ≥ the 3-position (relative to the O-function;
• R⁹ is an alkyl radical with 2-15 C-atoms which is either linear or has a branch;
• n = an integer between 1 and 4; and
• a + b ≥ 2 and also c + d ≤ 3, and a and also c can adopt any values from 0.01-0.8, and b and d can adopt any values from 0.1-1.99, wherein the content of dissolved aluminium relative to dissolved alkaline earth metal lies in the range between 0.2 and approximately 20 mol %.

2. Solutions according to claim 1, **characterised in that** the alkaline earth metal concentration preferably lies in the range of 0.4 to 1.6, particularly preferably between 0.7 to 1.4 mmol/g.

3. Solutions according to claim 1 or 2, **characterised in that** the room temperature viscosities are ≤ 300 cP, preferably ≤ 200 cP, particularly preferably ≤ 100 cP.

4. Solutions according to claim 1 to 3, **characterised in that** the contents of protic impurities, relative to dissolved alkaline earth element, generally lie between 0.1 and 40 mol %, preferably 1 and 30 mol %.

5. Solutions according to claim 1 to 4, **characterised in that** the content of dissolved aluminium relative to dissolved alkaline earth metal lies in the range between 1 and 8 mol %.

6. Solutions according to claim 1 to 5, **characterised in that** preferably a C₂-C₄ glycol monoether, for example 2-ethoxyethanol, 3-ethoxy-1-propanol, 3-ethoxy-1-butanol, 2-(2-ethylhexoxy)ethanol, 2-butoxyethanol, 2-hexyloxyethanol and also 1,3-propylene glycol monobutyl ether or any mixture of at least two of the alcohols listed is used as an alcohol containing an alkoxy function HO(CHR⁸)ₙOR⁹.

7. Solutions according to claim 1 to 6, **characterised in that** they contain 0.1 to 80 mol %, particularly preferably 1 to 40 mol %, free alcohol, relative to alkaline earth metal found in solution.

8. A method for the preparation of mixed alkaline earth alkoxide compounds M(OCH₂R⁶)₂₋ₓ(OR⁷)ₓ in aprotic solvents, **characterised in that** an alkaline earth metal activated by means of alkyl aluminium compounds is reacted in an aprotic solvent with an alcohol containing an alkoxy function HO(CHR⁸)ₙOR⁹ and also a primary alcohol that is unbranched or has a branch ≥ the 3-position and has 2-15 C-atoms HOR⁷ and, if applicable, a primary alcohol that is branched in the 2-position.

9. A method according to claim 8, **characterised in that** trialkyl, alkyl-alkoxy and/or alkyl-halide compounds are used as the alkyl aluminium compound.

10. A method according to claim 8 or 9, **characterised in that** hydrocarbons are used as the solvent, wherein either aliphatic solvents selected from the group consisting of cyclohexane, methylcyclohexane, hexane, heptane, octane, nonane, decane, dodecane, decalin and also commercially available boiling cuts (benzine fractions) or aromatic solvents selected from the group consisting of benzene, toluene, ethylbenzene, xylenes and also cumene are used.

11. A method according to claim 8 to 10, **characterised in that** the reaction is effected at temperatures between approximately 0 and 180 °C, preferably between approximately 40 and 140 °C, in particular at the boiling temperature of the solvent.

12. A method according to claim 8 to 11, **characterised in that** preferably a C₂-C₄ glycol monoether, for example 2-ethoxyethanol, 3-ethoxy-1-propanol, 3-ethoxy-1-butanol, 2-(2-ethylhexoxy)ethanol, 2-butoxyethanol, 2-hexyloxyethanol and also 1,3-propylene glycol monobutyl ether or any mixture of at least two of the alcohols listed is used as the alcohol containing an alkoxy function HO(CHR⁸)ₙOR⁹; isobutanol, 2-methyl-1-pentanol, 2-ethyl-1-butanol, 2-ethyl-1-pentanol, 2-ethyl-4-methyl-1-pentanol, 2-propyl-1-heptanol, 2-methyl-1-hexanol, 2-ethylhexanol and 2-ethyl-5-methyl-1-octanol or any mixture of at least two of the alcohols listed is used as the alcohol that is branched in the 2-position (HOCH₂R⁶); and ethanol, propanol, butanol, pentanol, hexanol, octanol, decanol, dodecanol, 3-methylbutan-1-ol or any mixture of at least two of the alcohols listed is used as the primary alcohol that is unbranched or has a branch ≥ the 3-position and has 2-15 C-atoms (HOR⁷).

13. A method according to claim 8 to 12, **characterised in that** in the first instance the alcohol (HOR⁷) is added, and only then are the alcohol containing an alkoxy function HO(CHR⁸)ₙOR⁹ and the alcohol that is branched in the 2-position (HOCH₂R⁶) added.

## Revendications

1. Solutions d'un mélange de composés de type alcoxyde de métal alcalino-terreux, de formule
M(OCH₂R⁶)_{2-a-b}(OR⁷)ₐ[O(CHR⁸)ₙOR⁹]_{b}
mélangés avec un composé de l'aluminium, de formule
Al(OCH₂R⁶)_{3-c-d}(OR⁷)_{c}[O(CHR⁸)ₙOR⁹]_{d}
dans un solvant aprotique, dans lesquelles formules
- M représente un atome d'un métal alcalino-terreux choisi parmi les magnésium, calcium, baryum et strontium ;
- le fragment symbolisé par OCH₂R⁶ est un groupe alcoxy comportant au moins 3 et au plus 40 atomes de carbone, ainsi qu'une ramification en position 2 par rapport à l'atome d'oxygène fonctionnel, c'est-à-dire que le groupe R⁶ a pour formule -CHR¹¹R¹¹ où R¹⁰ et R¹¹ représentent chacun, indépendamment l'un de l'autre, un groupe alkyle en C₁-C₁₈ ;
- R⁷ représente un groupe alkyle comportant de 2 à 15 atomes de carbone, soit linéaire, soit porteur d'une ramification en position 3 ou plus éloignée par rapport à l'atome d'oxygène fonctionnel ;
- R⁸ représente un groupe alkyle comportant de 1 à 6 atomes de carbone, soit linéaire, soit porteur d'une ramification en position 3 ou plus éloignée par rapport à l'atome d'oxygène fonctionnel ;
- R⁹ représente un groupe alkyle comportant de 2 à 15 atomes de carbone, soit linéaire, soit porteur d'une ramification ;
- l'indice n est un nombre entier valant de 1 à 4 ;
- et la somme des indices a + b est inférieure ou égale à 2, la somme des indices c + d est inférieure ou égale à 3, a et c peuvent prendre toute valeur de 0,01 à 0,8, et b et d peuvent prendre toute valeur de 0,1 à 1,99, étant entendu que le rapport de la quantité d'aluminium dissous à la quantité de métal alcalino-terreux dissous vaut de 0,2 à environ 20 % en moles.

2. Solutions conformes à la revendication 1, **caractérisées en ce que** la concentration de métal alcalino-terreux vaut de préférence de 0,4 à 1,6 mmol/g, et mieux encore de 0,7 à 1,4 mmol/g.

3. Solutions conformes à la revendication 1 ou 2, **caractérisées en ce que** la viscosité à température ambiante est inférieure ou égale à 300 cP, de préférence inférieure ou égale à 200 cP, et mieux encore inférieure ou égale à 100 cP.

4. Solutions conformes à l'une des revendications 1 à 3, **caractérisées en ce que** la teneur en impuretés protiques, rapportée à l'élément alcalino-terreux dissous, vaut en général de 0,1 à 40 % en moles, et de préférence de 1 à 30 % en moles.

5. Solutions conformes à l'une des revendications 1 à 4, **caractérisées en ce que** la teneur en aluminium dissous, rapportée au métal alcalino-terreux dissous, vaut de 1 à 8 % en moles.

6. Solutions conformes à l'une des revendications 1 à 5, **caractérisées en ce qu'**est utilisé, en tant qu'alcool comportant un groupe fonctionnel alcoxy, de formule HO(CHR⁸)ₙOR⁹, de préférence un monoéther de glycol en C₂-C₄, par exemple 2-éthoxy-éthanol, 3-éthoxypropan-1-ol, 3-éthoxy-butan-1-ol, 2-(2-éthyl-hexyloxy)-éthanol, 2-butoxy-éthanol, 2-hexyloxy-éthanol, ainsi que l'éther monobutylique de 1,3-propylèneglycol, ou un mélange quelconque d'au moins deux des alcools indiqués.

7. Solutions conformes à l'une des revendications 1 à 6, **caractérisées en ce qu'**elles contiennent de 0,1 à 80 % en moles et surtout de 1 à 40 % en moles d'alcool libre, par rapport au métal alcalino-terreux présent dans la solution.

8. Procédé de préparation de mélanges de composés de type alcoxyde de métal alcalino-terreux, de formule M(OCH₂R⁶)₂₋ₓ(OR⁷)ₓ, dans des solvants aprotiques, **caractérisé en ce qu'**on fait réagir, dans un solvant aprotique, un métal alcalino-terreux, activé au moyen d'un composé ou de composés de type alkyl-aluminium, avec un alcool comportant un groupe fonctionnel alcoxy, de formule HO(CHR⁸)ₙOR⁹, ainsi qu'avec un alcool primaire de formule HOR⁷, comportant de 2 à 15 atomes de carbone, non ramifié ou porteur d'une ramification en position 3 ou plus éloignée, et en option, avec un alcool primaire porteur d'une ramification en position 2.

9. Procédé conforme à la revendication 8, **caractérisé en ce que** l'on utilise, en tant que composé(s) de type alkyl-aluminium, un trialkyl-aluminium, un alcoxyde d'alkyl-aluminium et/ou un halogénure d'alkyl-aluminium.

10. Procédé conforme à la revendication 8 ou 9, caractérisé en que l'on utilise comme solvants des hydrocarbures qui sont soit des solvants aliphatiques choisis dans l'ensemble formé par les cyclohexane, méthyl-cyclohexane, hexane, heptane, octane, nonane, décane, dodécane et décaline, ainsi que les coupes de distillation commercialisées (fractions de type essence), soit des solvants aromatiques choisis dans l'ensemble formé par les benzène, toluène, éthyl-benzène, xylènes et cumène.

11. Procédé conforme à l'une des revendications 8 à 10, **caractérisé en ce qu'**on effectue la réaction à une température d'environ 0 à 180 °C, et de préférence d'environ 40 à 140 °C, et en particulier, à la température d'ébullition du solvant.

12. Procédé conforme à l'une des revendications 8 à 11, **caractérisé en ce qu'**on utilise, en tant qu'alcool comportant un groupe fonctionnel alcoxy, de formule HO(CHR⁸)ₙOR⁹, de préférence un monoéther de glycol en C₂-C₄, par exemple 2-éthoxy-éthanol, 3-éthoxypropan-1-ol, 3-éthoxy-butan-1-ol, 2-(2-éthyl-hexyloxy)-éthanol, 2-butoxy-éthanol, 2-hexyloxy-éthanol, ainsi que l'éther monobutylique de 1,3-propylèneglycol, ou un mélange quelconque d'au moins deux des alcools indiqués ; en tant qu'alcool primaire porteur d'une ramification en position 2, de formule HOCH₂R⁶, l'un des isobutanol, 2-méthylpentan-1-ol, 2-éthyl-butan-1-ol, 2-éthyl-pentan-1-ol, 2-éthyl-4-méthylpentan-1-ol, 2-propyl-heptan-1-ol, 2-méthyl-hexan-1-ol, 2-éthyl-hexan-1-ol et 2-éthyl-5-méthyl-octan-1-ol, ou un mélange quelconque d'au moins deux des alcools indiqués ; et en tant qu'alcool primaire de formule HOR⁷, comportant de 2 à 15 atomes de carbone et non ramifié ou porteur d'une ramification en position 3 ou plus éloignée, l'un des éthanol, propanol, butanol, pentanol, hexanol, octanol, décanol, dodécanol et 3-méthyl-butan-1-ol, ou un mélange quelconque d'au moins deux des alcools indiqués.

13. Procédé conforme à l'une des revendications 8 à 12, **caractérisé en ce qu'**on ajoute d'abord l'alcool de formule HOR⁷, et ensuite seulement l'alcool comportant un groupe fonctionnel alcoxy, de formule HO(CHR⁸)ₙOR⁹, et l'alcool primaire porteur d'une ramification en position 2, de formule HOCH₂R⁶.
